(19) Europäisches Patentamt
European Patent Office
Office européen des brevets

(11) **EP 1 470 417 B1**

(12) **EUROPÄISCHE PATENTSCHRIFT**

(45) Veröffentlichungstag und Bekanntmachung des Hinweises auf die Patenterteilung:
**11.03.2009 Patentblatt 2009/11**

(51) Int Cl.:
*G01N 33/00* (2006.01)  *F24F 6/02* (2006.01)

(21) Anmeldenummer: **03743272.1**

(22) Anmeldetag: **03.01.2003**

(86) Internationale Anmeldenummer:
**PCT/EP2003/000033**

(87) Internationale Veröffentlichungsnummer:
**WO 2003/081231 (02.10.2003 Gazette 2003/40)**

(54) **VERFAHREN UND VORRICHTUNG ZUR KALIBRIERUNG EINES FEUCHTESENSORS SOWIE SENSORANORDNUNG MIT EINEM KALIBRIERBAREN FEUCHTESENSOR**

METHOD AND DEVICE FOR CALIBRATING A HUMIDITY SENSOR AND SENSOR ARRANGEMENT COMPRISING A HUMIDITY SENSOR THAT CAN BE CALIBRATED

PROCEDE ET DISPOSITIF DE CALIBRAGE D'UN DETECTEUR D'HUMIDITE ET SYSTEME DETECTEUR DOTE D'UN DETECTEUR D'HUMIDITE CALIBRABLE

(84) Benannte Vertragsstaaten:
**AT BE BG CH CY CZ DE DK EE ES FI FR GB GR HU IE IT LI LU MC NL PT SE SI SK TR**

(30) Priorität: **30.01.2002 DE 10203637**

(43) Veröffentlichungstag der Anmeldung:
**27.10.2004 Patentblatt 2004/44**

(73) Patentinhaber: **Testo AG**
**79853 Lenzkirch (DE)**

(72) Erfinder:
• **ROMBACH, Martin**
**79853 Lenzkirch (DE)**
• **LANGENBACHER, Markus**
**79104 Freiburg (DE)**

(74) Vertreter: **Schmuckermaier, Bernhard**
**Westphal, Mussgnug & Partner**
**Patentanwälte**
**Herzog-Wilhelm-Strasse 26**
**80331 München (DE)**

(56) Entgegenhaltungen:
**DE-A- 3 411 306          DE-A- 3 936 138**
**FR-A- 2 716 975          US-A- 6 073 480**
**US-B1- 6 299 147**

EP 1 470 417 B1

**Beschreibung**

Die vorliegende Erfindung bezieht sich auf ein Verfahren zur Kalibrierung eines Feuchtesensors, bei dem mit dem Feuchtesensor unter ersten Umgebungsbedingungen und unter zweiten, von den ersten verschiedenen Umgebungsbedingungen jeweils ein Feuchtewert erfaßt wird und bei dem aus den Parametern der Umgebungsbedingungen und den gemessenen Feuchtewerten ein Korrekturwert für den Feuchtesensor bestimmt wird.

Mit Hilfe von Feuchtesensoren wird in offenen oder geschlossenen Gasvolumina die relative Feuchte gemessen, das heißt die Menge an Feuchtigkeit, die in dem jeweiligen Gas gelöst ist relativ zu der maximal bei der gegebenen Temperatur in dem Gas lösbaren Feuchtigkeitsmenge.

Solche Messungen sind beispielsweise dem Durchschnittsverbraucher als Messungen der relativen Luftfeuchtigkeit zur Bestimmung der klimatischen Bedingungen geläufig, sie sind jedoch auch im industriellen Umfeld beispielsweise bei der Bestimmung der relativen Luftfeuchte in Druckgasbehältern üblich.

Feuchtesensoren werden oft durch physikalische und chemische Einflüsse stark belastet und können dadurch ihre Ansprechcharakteristik gegenüber einer anfänglichen

Kalibrierung signifikant verändern. Daher ist in gewissen Abständen eine Nachkalibrierung notwendig.

Aus dem Stand der Technik (DE 3936138 A1, US 6073480) ist eine Kalibrierung eines Feuchtesensors dadurch bekannt, daß eine erste Feuchtemessung bei einer ersten Temperatur und danach bei einer zweiten Temperatur eine zweite Feuchtemessung durchgeführt wird, wobei der Gasdruck bei beiden Messungen gleichbleibt. Durch die gemessenen Temperatur- und Feuchtigkeitswerte ist eine Berechnung eines Korrekturwertes für die Feuchtemessung möglich, da der mathematische Zusammenhang zwischen Temperatur und tatsächlicher relativer Feuchte bekannt ist.

Es ist jedoch zur Anwendung dieser Technik notwendig, einerseits Temperatursensoren im Bereich der Feuchtsensoren anzuordnen, und andererseits Heiz- oder Kühlelemente vorzusehen. Außerdem erfordert eine derartige Kalibrierung Zeit zur Einstellung der jeweiligen Meßtemperaturen.

Als weiterer Stand der Technik wird die FR 2 716 975 A genannt.

In diesem Dokument wird ein Verfahren zur Kalibrierung eines Feuchtesensors beschrieben, bei dem unter verschiedenen Druckbedingungen und sonst gleichen Umgebungsbedingungen zwei Feuchtewerte erfasst werden und aus den Druckwerten und den gemessenen Feuchtewerten ein Korrekturwert bestimmt wird.

Der vorliegenden Erfindung liegt demgegenüber die Aufgabe zugrunde, ein Verfahren zur Kalibrierung eines Feuchtesensors sowie eine entsprechende Vorrichtung und eine Sensoranordnung mit einem kalibrierbaren Feuchtsensor der eingangs genannten Art zu schaffen, bei dem der Kalibriervorgang vereinfacht ist und möglichst geringe Hilfsmittel zu seiner Durchführung benötigt werden.

Die Aufgabe wird erfindungsgemäß dadurch gelöst, daß bei einem ersten Druck P1 und bei einem zweiten, vom ersten unterschiedlichen Druck P2 und bei sonst gleichen Bedingungen jeweils ein Feuchtewert U1, U2 erfaßt wird, wobei zumindest das Verhältnis des ersten Druckwertes P1 und des zweiten Druckwertes P2 bekannt ist und daß der Korrekturwert für den Feuchtesensor aus dem Verhältnis der Druckwerte und den gemessenen Feuchtigkeitswerten bestimmt wird.

Die Aufgabe wird außerdem gelöst durch eine Vorrichtung mit einem Drucksensor, einer Eingabeeinrichtung zur Eingabe der Meßwerte des Feuchtesensors in einen ersten Datenspeicher und einer Recheneinrichtung, die aus dem Verhältnis zweier Druckwerte und zwei bei den jeweiligen Druckwerten unter ansonsten gleichen Bedingungen erfaßter Feuchtewerte einen Korrekturwert des Feuchtesensors bestimmt.

Wird der Feuchtewert bei zwei unterschiedlichen Drücken gemessen, wobei die Temperatur und andere Umgebungsbedingungen konstant gehalten werden und wobei auch die gleiche Feuchtigkeitsmenge absolut in dem Gas gelöst bleibt, so ändert sich der Wert der relativen Feuchte zwischen den beiden Messungen. Aus dem Verhältnis der beiden Druckwerte kann auf das Verhältnis der beiden real vorliegenden Feuchtigkeitswerte der relativen Gasfeuchtigkeit geschlossen werden. Stimmen die Verhältnisse der Drücke und der gemessenen relativen Gasfeuchten nicht überein, so kann aus der Abweichung der Korrekturwert bestimmt werden, der bei der jeweiligen Feuchtemessung anzusetzen ist. Dieser Korrekturwert ist dann von dem gemessenen Wert der relativen Feuchte abzuziehen oder zu diesem zu addieren.

Die Vorrichtung zur Kalibrierung eines Feuchtesensors sieht demgemäß einen Drucksensor vor, wenn dieser in der Anlage im Bereich des Feuchtesensors nicht ohnehin vorgesehen ist sowie eine Eingabeeinrichtung, über die der Kalibriervorrichtung die gemessenen Feuchtewerte des Feuchtesensors zugeführt werden können. Eine Recheneinrichtung der Kalibriervorrichtung bestimmt dann aus den gemessenen Daten den Korrekturwert und gibt diesen aus.

Vorteilhaft wird das Verfahren zur Kalibrierung derart durchgeführt, daß der von einem gemessenen Feuchtewert zu subtrahierende Korrekturfaktor k bestimmt wird nach der Gleichung:

$$k = ((P1/P2) * U2 - U1)/(P1/P2-1).$$

Idealerweise gilt bei einem Gas gleichbleibender Konsistenz und konstanter Temperatur, daß das Verhältnis der beiden Druckwerte, bei denen gemessen wird, dem Verhältnis der tatsächlich vorliegenden relativen Luftfeuchten entspricht:

$$P1/P2 = U1 \ (real)/U2 \ (real).$$

Da die gemessenen Feuchtewerte U1, U2 mit den realen Feuchtewerten vor der Kalibrierung nicht übereinstimmen, berechnet sich der reale Feuchtewert

$$U1 \ (real) = U1 - k \ \text{sowie} \ U2 \ (real) = U2 - k.$$

Es ergibt sich die Gleichung: P1/P2 = (U1 - k)/(U2 - k).
Diese Gleichung nach k aufgelöst ergibt:

$$k = ((P1/P2)*U2 - U1)/(P1/P2 - 1).$$

Dies gilt unter der Annahme, daß k unabhängig vom Wert von U ist. Durch das erfindungsgemäße Kalibrierverfahren läßt sich somit der Feuchtesensor unter Verwendung eines Drucksensors kalibrieren, ohne daß die tatsächlich vorliegende Feuchte direkt bestimmt wird. Es ist lediglich notwendig, einen kalibrierten Drucksensor zu verwenden, was jedoch dadurch vereinfacht ist, daß Drucksensoren in ihrer Kalibrierung relativ stabil sind.
Auf praktische Weise wird das Verfahren zur Kalibrierung eines Feuchtesensors in einer druckbeaufschlagbaren gasgefüllten Anlage vorteilhaft derart durchgeführt, daß mittels eines an der Anlage vorgesehenen Ventils Gas abgelassen oder zugeführt wird, und daß Druckwerte und Feuchtewerte vor und nach dem Ablassen / Zuführen von Gas registriert werden, wobei jeweils vor der Feuchtemessung ein Temperaturausgleich abgewartet wird.
An druckbeaufschlagbaren gasgefüllten Anlagen sind typischerweise Ventile zum Befüllen mit Gas oder zum Ablassen von Gas ohnehin vorgesehen. Über ein derartiges Ventil kann entweder Gas abgelassen oder zugeführt werden, wobei abgelassenes Gas in einem externen Drucktank gespeichert werden kann. Vor- und nach dem Ablassen beziehungsweise Zuführen von Gas werden jeweils Feuchtemessungen durchgeführt, wobei nach dem Ablassen oder Zuführen von Gas ein Temperaturausgleich abgewartet werden muß, da beide Feuchtemessungen bei derselben Temperatur stattfinden müssen und da durch die Entspannung des Gases beziehungsweise durch die Druckerhöhung eine Temperaturerniedrigung beziehungsweise -erhöhung zu erwarten ist. Anstelle von Ventilen können auch Druckminderer eingesetzt werden.
Zur Kalibrierung des Feuchtesensors kann dieser in einem kleinen, druckdicht abgeschlossenen Teil (Meßkammer) der Anlage betrieben werden, so daß die unterschiedlichen Druckwerte nicht eine Druckänderung in der gesamten Anlage erfordern, sondern nur der Gasdruck in dem Teilvolumen geändert werden muß, in dem der Feuchtesensor angeordnet ist. Die entsprechenden Drucksensoren können in der Anlage im Bereich des Feuchtesensors ohnehin vorgesehen sein, sie können jedoch auch im Zuge der Kalibrierung in das Volumen eingebracht werden oder der jeweilige Druck kann beim Ablassen oder Zuführen von Gas in der jeweiligen Zuleitung außerhalb der Anlage gemessen werden. Somit kann die Kalibrierung ohne größere Änderungen oder Installationen an der druckbeaufschlagbaren gasgefüllten Anlage von Servicepersonal einfach, zuverlässig und schnell durchgeführt werden. Die Meßkammer mit eingebautem Feuchtesensor ist also durch geeignete Mittel (Ventil, Druckminderer etc.) dazu ausgelegt, die für das Kalibrierverfahren erforderlichen zwei Drücke für den Feuchtesensor zu erzeugen.
Die Erfindung bezieht sich außerdem auf eine Sensoranordnung mit einem nach dem Verfahren gemäß einem der Ansprüche 1 bis 3 kalibrierbaren Feuchtesensor und mit einer Korrektureinrichtung mit einem Speicher, in dem ein von dem gemessenen Feuchtewert zu subtrahierender Korrekturwert speicherbar ist, wobei die Korrektureinrichtung von dem erfaßten Meßwert der Feuchte den Korrekturwert subtrahiert und insbesondere das Ergebnis einer Anzeigeeinrichtung zuleitet.
Die Sensoranordnung weist somit eine Korrektureinrichtung 8 auf, durch die nach einer Kalibrierung der von dem Feuchtesensor erfaßte Feuchtewert mittels des Korrekturwertes korrigiert wird, so daß der tatsächlich vorliegende Feuchtewert als korrigierter Meßwert zum Ablesen oder zur Weiterverarbeitung, beispielsweise in einer Meßwarte zur Verfügung steht.

Im folgenden wird die Erfindung anhand einer Zeichnung beispielhaft gezeigt und nachfolgend erläutert. Dabei zeigt:

Figur 1     in einer schematischen Darstellung einen Feuchtesen- sor sowie eine Vorrichtung zu seiner Kalibrierung,

Figur 2     schematisch die Funktion der Recheneinrichtung zur Ermittlung des Korrekturwertes,

Figur 3     ein Ablaufdiagramm des Kalibriervorgangs.

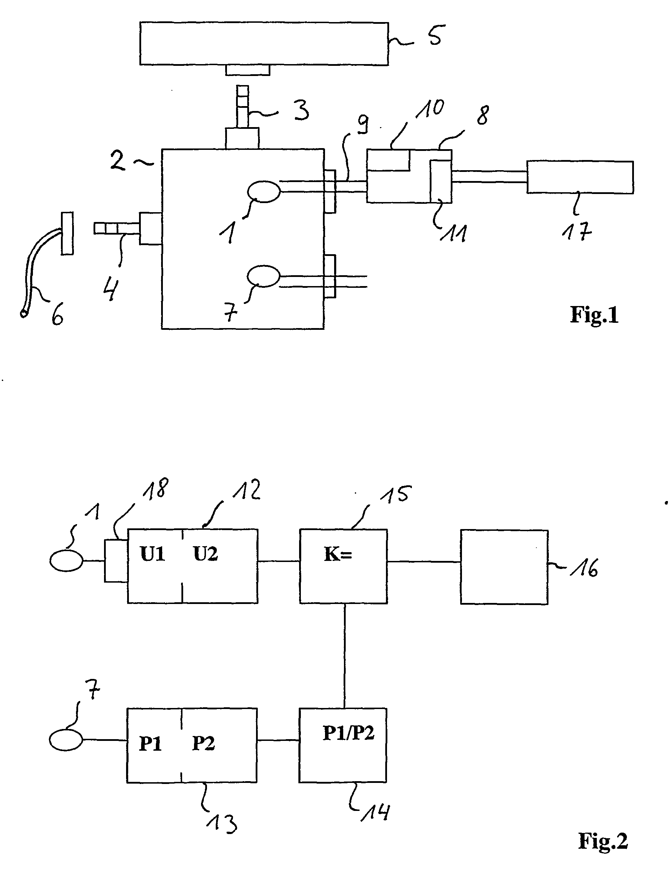

Figur 1 zeigt einen Feuchtesensor 1 in einer gasdichten Meßkammer 2. Die Meßkammer 2 weist einen ersten Gasanschluß 3 und einen zweiten Gasanschluß 4 auf, mittels deren die Meßkammer mit einer gasgefüllten druckbeaufschlagbaren Anlage 5 einerseits und mit einem weiteren Gasraum mittels eines Druckschlauchs 6 andererseits verbunden werden kann.

In der Meßkammer 2 ist außerdem ein Drucksensor 7 vorgesehen. Dieser Drucksensor kann ein handelsüblicher Drucksensor sein, er sollte jedoch kalibriert sein und über eine möglichst hohe Meßgenauigkeit verfügen.

Um beispielsweise die Gasfeuchte in der druckbeaufschlagbaren Anlage 5 zu messen und gleichzeitig den Feuchtesensor 1 zu kalibrieren, wird zunächst die Meßkammer 2 über den ersten Gasanschluß 3 mit der druckbeaufschlagbaren Anlage 5 verbunden, so daß ein Gasaustausch und Druckausgleich stattfinden kann. Danach wird mittels des Drucksensors 7 der Gasdruck in der Meßkammer gemessen und die Meßkammer 2 von der druckbeaufschlagbaren Anlage abgetrennt. Außerdem wird unter den nun vorliegenden Bedingungen mittels des Feuchtesensors 1 der erste Feuchtewert gemessen. Dabei wird die Korrektureinrichtung 8, die mit den Zuleitungen 9 des Feuchtesensors verbunden ist, und die im Normalbetrieb den im Speicher 10 gespeicherten Korrekturwert von dem unmittelbaren Meßwert des Feuchtesensors 1 subtrahiert, zur Kalibrierung überbrückt.

Danach wird mittels des zweiten Gasanschlusses 4 über den Druckschlauch 6 eine gewisse Gasmenge aus der Meßkammer 2 abgelassen, so daß der Gasdruck abgesenkt wird. Danach wird die Meßkammer 2 wieder gasdicht verschlossen.

Nachdem die Temperatur in der Meßkammer 2 sich normalisiert hat, wird nun ein zweiter Feuchtewert U2 sowie ein zweiter Druckwert P2 gemessen. Aus den beiden gemessenen Druckwerten

sowie den beiden gemessenen und unkorrigierten Feuchtemeßwerten kann dann ein Korrekturwert ermittelt werden, der in dem Speicher 10 abgelegt wird. Bei folgenden Feuchtemessungen wird dann innerhalb der Korrektureinrichtung 8 der im Speicher abgelegte Korrekturwert von dem durch den Feuchtesensor 1 gemessenen Feuchtewert in einer Subtraktionseinheit 11 abgezogen und als korrigierter Meßwert mittels einer Anzeige 17 ausgegeben.

In der Figur 2 sind schematisch die Erfassungs- und Rechenschritte bei einem Kalibriervorgang dargestellt. Mittels des Feuchtesensors 1 wird in einem ersten Schritt der Feuchtewert U1 erfaßt. Gleichzeitig wird mittels des Drucksensors 7 der Druckwert P1 erfaßt. Beide Meßwerte werden in einem ersten Datenspeicher 12 und einem zweiten Datenspeicher 13 hinterlegt.

In einem zweiten Schritt wird dann bei abgesenktem Druck und ansonsten gleichen Umgebungsbedingungen ein zweiter Feuchtewert U2 sowie ein zweiter Druckwert P2 gemessen. Beide Meßwerte werden wieder in den entsprechenden ersten und zweiten Datenspeichern 12, 13 hinterlegt.

Es wird danach das Verhältnis P1/P2 berechnet und in einem dritten Datenspeicher 14 hinterlegt. In einer Recheneinrichtung 15 wird dann mittels eines Mikroprozessors k nach der im Patentanspruch 2 angegebenen Formel berechnet und an eine Ausgabeeinrichtung 16 weitergegeben, die den Korrekturwert beispielsweise an die Korrektureinrichtung 8 weitergibt, wo dieser im Speicher 10 gespeichert wird.

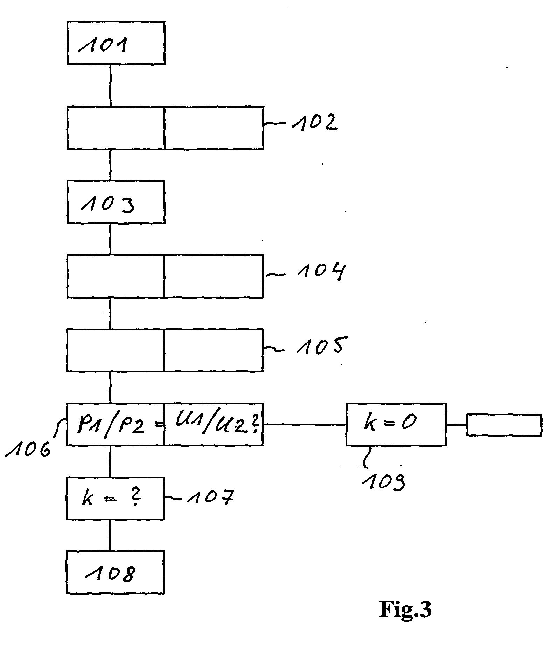

In der Figur 3 ist der Kalibriervorgang nochmals in Form von Ablaufschritten dargestellt. In einem ersten Schritt 101 wird in der Meßkammer ein bestimmter Druck eingestellt. Im zweiten Schritt 102 wird der Druckwert P1 gemessen sowie der Feuchtewert U1. Im dritten Schritt 103 wird der Druck in der Meßkammer beispielsweise durch Ablassen von Gas geändert. Im vierten Schritt 104 wird ein zweiter Druck P2 sowie ein zweiter Feuchtewert U2 gemessen. Im fünften Schritt 105 wird der Quotient aus P1 und P2 sowie der Quotient aus U1 und U2 berechnet. Im sechsten Schritt 106 werden die Quotienten aus P1 und P2 sowie U1 und U2 miteinander verglichen. Stimmen die beiden Quotienten überein, so wird im nächsten Schritt (109) der Korrekturwert k = 0 gesetzt und das Kalibrierverfahren beendet. Stimmen die Quotienten nicht überein, so wird im nächsten Schritt 107 k aus der im Patentanspruch 2 angegebenen Formel berechnet. Im achten Schritt 108 wird dann k im Speicher 10 zur Korrektur folgender Feuchtemessungen gespeichert.

Bei einer Beispielkalibrierung wurde zunächst ein erster Druckwert P1 = 4,5 bar gemessen. Bei diesem Druckwert wurde mittels des Feuchtesensors ein Feuchtewert U1 = 1,7% relative Feuchte gemessen. Bei einem zweiten Gasdruck wurde der Druckwert P2 = 0,9 bar und ein Feuchtewert von 0,5% relativer Feuchte gemessen. Als Quotient der Druckwerte ergab sich 5. Damit müßte der relative Feuchtewert bei hohem Druck von 1,7% relativer Feuchte auf 0,34% relativer Feuchte bei dem niedrigeren Druckwert absinken. Gemessen wurde aber ein Wert von 0,5% relativer Feuchte bei dem niedrigeren Druckwert. Das heißt, es ist eine Korrektur notwendig, k ergibt sich wie folgt

$$k = (5*0,5\% \text{ r. F} - 1,7\% \text{ r. F})/(5 - 1) = 0,2\% \text{ r. F.}$$

Die korrigierten Feuchtewerte gemäß der durchgeführten Messung sind dann U1 (real) = 1,7% r. F - 0,2% r. F = 1,5% r. F und

$$U2 \text{ (real)} = 0,5\% \text{ r. F} - 0,2\% \text{ r. F} = 0,3\% \text{ r. F.}$$

Das beschriebene Kalibrierverfahren kann während des Betriebs des Feuchtesensors zyklisch beziehungsweise periodisch durchgeführt und jeweils ein neuer Korrekturwert berechnet und gespeichert werden.

Das erfindungsgemäße Verfahren kann neben den gezeigten Ausgestaltungen auf vielfältige Art und Weise realisiert werden. Insbesondere gibt es zahlreiche Ausführungsformen von Vorrichtungen zur Durchführung des erfindungsgemäßen Verfahrens.

**Patentansprüche**

1. Verfahren zur Kalibrierung eines Feuchtesensors (1), bei dem mit dem Feuchtesensor (1) unter ersten Umgebungsbedingungen und unter zweiten, von den ersten verschiedenen Umgebungsbedingungen jeweils ein Feuchtewert (U1, U2) erfasst wird und bei dem aus den Parametern der Umgebungsbedingungen und den gemessenen Feuchtewerten ein Korrekturwert für den Feuchtesensor bestimmt wird, wobei bei einem ersten Druck (P1) und bei einem zweiten, vom ersten unterschiedlichen Druck (P2) und bei sonst gleichen Bedingungen jeweils ein Feuchtewert (U1, U2) erfasst wird, wobei zumindest das Verhältnis des ersten Druckwertes (P1) und des zweiten Druckwertes (P2) bekannt ist, der Korrekturwert für den Feuchtesensor (1) aus dem Verhältnis der Druckwerte und den gemessenen Feuchtigkeitswerten (U1, U2) bestimmt wird, und der von einem gemessenen Feuchtewert zu subtrahierende Korrekturfaktor k bestimmt wird nach der Gleichung:

$$k= ((P1/P2)*U2 - U1)/(P1/P2 - 1).$$

2. Verfahren nach Anspruch 1 zum Kalibrieren eines Feuchtesensors (1) in einer druckbeaufschlagbaren gasgefüllten Anlage (5),
   **dadurch gekennzeichnet, dass** mittels eines an der Anlage vorgesehenen Ventils (3, 4) Gas abgelassen oder zugeführt wird, und dass Druckwerte und Feuchtewerte vor und nach dem Ablassen / Zuführen von Gas registriert werden, wobei jeweils vor der Feuchtemessung ein Temperaturausgleich abgewartet wird.

**Claims**

1. Method for calibrating a humidity sensor (1), in which a respective humidity value (U1, U2) is detected by the humidity sensor (1) under first ambient conditions and under second ambient conditions different from the first, and in which a correction value for the humidity sensor is determined from the parameters of the ambient conditions and the measured humidity values, wherein a respective humidity value (U1, U2) is detected at a first pressure (P1) and at a second pressure (P2) different from the first and under otherwise identical conditions, wherein at least the ratio of the first pressure value (P1) and second pressure value (P2) is known, the correction value for the humidity sensor (1) is determined from the ratio of the pressure values and the measured humidity values (U1, U2), and the correction factor k to be subtracted from a measured humidity value is determined according to the equation:

$$k = ((P1/P2)*U2 - U1)/(P1/P2 - 1).$$

**2.** Method according to claim 1 for calibrating a humidity sensor (1) in a pressurisable gas-filled installation (5), **characterised in that** gas is discharged or supplied by means of a valve (3, 4) provided on the installation, and **in that** pressure values and humidity values are recorded before and after the discharge/supply of gas, wherein in each case there is a wait for a temperature equalisation before measuring the humidity.

**Revendications**

**1.** Procédé de calibrage d'un détecteur d'humidité (1) selon lequel :

on saisit respectivement avec le détecteur d'humidité (1) une valeur d'humidité (U1) dans des premières conditions ambiantes ainsi qu'une valeur d'humidité (U2) dans des secondes conditions ambiantes différentes des premières, et à partir des paramètres des conditions ambiantes et des valeurs d'humidité mesurées, on détermine une valeur de correction du détecteur d'humidité,
on saisit une valeur d'humidité (U1), à une première pression (P1) ainsi qu'une valeur d'humidité (U2) à une seconde pression (P2), différente de la première, les autres conditions étant par ailleurs identiques, et connaissant au moins le rapport entre la première valeur de pression (P1) et la seconde valeur de pression (P2), on détermine la valeur de correction du détecteur d'humidité (1) à partir du rapport des valeurs de pression et des valeurs d'humidité (U1, U2) mesurées, puis on détermine le coefficient de correction k à retrancher de la valeur d'humidité mesurée selon l'équation suivante :

$$k = ((P1/P2) * U2 - U1) / (P1/P2 - 1) .$$

**2.** Procédé selon la revendication 1 de calibrage d'un détecteur d'humidité (1) dans une installation remplie de gaz mis en pression, **caractérisé en ce qu'**
à l'aide d'une vanne (3, 4) de l'installation, on évacue ou on introduit du gaz et on enregistre les valeurs de pression et d'humidité avant ou après l'évacuation/introduction de gaz, et avant chaque mesure de l'humidité, on attend l'équilibre des températures.

Fig.1

Fig.2

101

102

103

104

105

$P_1/P_2 = U_1/U_2$ ?    $k = 0$

106                      109

$k = ?$  107

108

**Fig.3**

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- DE 3936138 A1
- US 6073480 A
- FR 2716975 A